# EUROPEAN PATENT APPLICATION

(11) **EP 4 755 357 A1**
(43) Date of publication of application: **10.06.2026**
(21) Application number: 24217526.3
(22) Date of filing: 04.12.2024
(51) Int. Cl.: A61F 13/15, A61F 13/496, A61F 13/49

(54) **APPARATUS AND METHOD FOR FORMING TORSO ENCIRCLING PORTIONS FOR PANT-STYLE DIAPER PRODUCTS**

(71) Applicant: Curt G. Joa, Inc., Sheboygan Falls, Wisconsin 53085-0903 (US)
(72) Inventor: SCHWARTZ, Christopher A., Howards Grove, WI 53083 (US); FRITZ, Jeffrey W., Plymouth, WI 53073 (US); SCHUETTE, David E., Sheboygan, WI 53083 (US)
(74) Representative: Potter Clarkson

(57) **Abstract**

Apparatus and method for manufacturing torso encircling portions for pant-style diaper products. The apparatus includes various assemblies for feeding, slitting, and welding continuous webs of material to create torso encircling portions with waist and leg elastic strands. The welded webs are slit into two portions, which are spread via a web guide assembly so the positions thereof are swapped in a cross-machine direction. Waist elastic strands are secured using an adhesive-free welding unit, while leg elastic strands are applied non-linearly using adhesive. This method reduces material costs and waste, providing a more comfortable and less bulky product. The apparatus also allows for the integration of elasticated waist and leg portions without additional layers, enhancing the fit and comfort of the final product.

## Description

The invention relates to apparatus for manufacturing torso encircling portions for use in the manufacture of pant-style diaper products. More specifically, the invention relates to an apparatus for manufacturing two-ply torso encircling portions having waist elastic strands secured by means of an adhesive-free welding unit and non-linearly applied leg elastic strands by means of adhesive. The invention also relates to a method for manufacturing such torso encircling portions.

Wearable absorbent products including, for example, adult incontinence products, enuresis pants, training pants and other pant-style diapers typically incorporate elasticated torso encircling portions intended to extend around a wearer's waist and keep the article snug against the wearer whilst being worn. Elasticated sections are preferably provided around the leg openings to hold the article snug against a wearer's legs and prevent leakage of urine.

The torso encircling portions may incorporate waist elastic strands, sandwiched under tension between inner and outer layers and arranged to encircle a wearer's waist to provide the required stretch, with leg elastic strands similarly sandwiched under tension between inner and outer layers around the leg openings.

Conventionally, the waist and leg elastic strands are secured in position between inner and outer layers of torso encircling portions by means of adhesive.

The adhesive used in the manufacture of such torso-encircling portions of pant-style diapers is, however, relatively expensive as a result of the cost of the adhesive and the need for complex delivery systems to apply the adhesive during manufacture.

Whilst welding has been used as an alternative to secure waist elastic strands between inner and outer layers, the need for a grooved surface to locate the elastic strands relative to one or more welds means that welding is better suited to securing elastic strands that are applied in straight lines as opposed to non-linearly applied elastic strands, such as those required to form elasticated leg openings. As a result, welding does not provide a reliable alternative to adhesive for securing leg elastic strands.

According to a first aspect of the invention there is provided an apparatus for manufacturing torso encircling portions of a pant-style diaper product, the apparatus comprising:
a first web infeed assembly to feed a first continuous web generally in a first feed direction, the first continuous web defining first and second back sheet waist sections and a back sheet leg section extending side by side in the first feed direction with the second back sheet waist section located between the first back sheet waist section and the back sheet leg section;
a second web infeed assembly to feed a second continuous web generally in a second feed direction, the second continuous web defining first and second top sheet sections extending side by side in the second feed direction;
a first slitting assembly configured to slit the second continuous web along a first cut line extending in the second feed direction to separate the first and second top sheet sections;
a waist elastic infeed configured to insert waist elastic strands under tension in the first feed direction on to the first and second back sheet waist sections of the first continuous web;
a waist top sheet infeed configured to direct the first top sheet section to overlie the first and second back sheet waist sections and sandwich the waist elastic strands therebetween;
a welding assembly configured to weld the first top sheet section and the first and second back sheet waist sections together intermittently in the first feed direction so as to entrap and secure the waist elastic strands therebetween and form a welded structure;
a second slitting assembly configured to slit the welded structure along a second cut line extending in the first feed direction to separate the welded structure along a juncture between the first and second back sheet waist sections of the first continuous web and thereby define first and second welded strips, the first welded strip including the first back sheet waist section, a first portion of the first top sheet section and waist elastic strands secured therebetween, and the second welded strip including the second back sheet waist section, the back sheet leg section, a second portion of the first top sheet section overlying the second back sheet waist section and waist elastic strands secured between the second back sheet waist section and the second portion of the first top sheet section;
a web guide assembly arranged to spread the first and second welded strips so that positions of the second back sheet waist section and the back sheet leg section extending in a third feed direction are swapped relative to the first back sheet waist section in a cross-machine direction such that the back sheet leg section is located between the first and second back sheet waist sections;
a leg elastic infeed assembly to insert leg elastic strands in a non-linear pattern in the third feed direction on to the back sheet leg section of the first continuous web; and
a leg top sheet infeed to direct the second top sheet section to overlie the back sheet leg section and sandwich the leg elastic strands therebetween.

The use of welding in the waist sections provides a clean and simple mechanism when compared with the use of adhesive, and results in a construction that is less sensitive to temperature and softer in feel because the elastic strands are entrapped by means of welds between top sheet and back sheet sections as opposed to being bonded in place by means of adhesive.

As outlined above, welding requires the use of a grooved surface to locate elastic strands relative to one or more welds, rendering it particularly suitable to secure and fix waist elastic strands inserted in straight lines. The resultant welds entrap the waist elastic strands securely between the top sheet and back sheet waist sections, thus eliminating the need to fold the back sheet waist sections, or the top sheet waist sections, along their outer edges to secure the outermost edge of the resultant waist portions. As a result, the use of welding to secure and fix waist elastic strands between the top sheet and back sheet sections further assists in the reduction of the amount of top sheet and back sheet material required to manufacture the torso encircling portions.

Preferably, the waist elastic infeed includes one or more waist elastic feed elements to insert the waist elastic strands to extend in substantially straight lines in the first feed direction on to the first and second back sheet waist sections with adjacent strands being spaced from each other in the cross-machine direction in order to provide a desired stretch effect.

In particularly preferred embodiments, the waist feed element(s) insert the waist elastic strands equidistantly spaced from each other in a cross-machine direction so as to provide unform stretch across the waist sections.

In order to produce a non-linear pattern of leg elastic on the back sheet leg section, the leg elastic infeed assembly may include one or more reciprocating leg elastic feed elements to insert the leg elastic strands so as to extend in a periodic wave pattern, or other non-linear pattern as may be desired, in the third feed direction on to the back sheet leg section. The creation of a periodic wave pattern allows leg openings to be cut at regular intervals in the torso encircling portions.

In alternative embodiments, a non-linear pattern of leg elastic on the back sheet leg section may be achieved by means of a leg elastic infeed assembly including a reciprocating drive element to drive movement of the back sheet leg section relative to one or more leg elastic feed elements to insert the leg elastic strands in a periodic wave pattern, or other non-linear pattern as may be desired, in the third feed direction on to the back sheet leg section. In preferred embodiments, the apparatus further includes an adhesive assembly configured to apply adhesive to one or both of the back sheet leg section and the leg elastic strands to adhere the leg elastic strands in the non-linear pattern on the back sheet leg section and the second top sheet section.

Apparatus according to such embodiments of the invention leverages the advantages of welding while mitigating against the associated costs associated with the use of adhesive.

More specifically, the apparatus facilitates the manufacture of a two-layer construction across waist and leg sections of torso encircling portions in which waist elastic strands are entrapped by means of welding in waist sections, and non-linearly applied leg elastic strands are secured by means of adhesive in leg sections between top sheet and back sheet sections. Accordingly, rather than attaching separate, elasticated leg portions, the relative arrangement of the components makes it possible to combine the use of welding and adhesive in the same apparatus and create elasticated leg portions integrally with elasticated waist portions whilst avoiding the need for additional layers of web material. This leads to torso encircling portions that are less bulky, allowing for a more discrete fitment.

Conventionally, it has only been possible to separate the use of welding and adhesive through the use of additional layers so that welding occurs between a back layer and a first top layer, and adhesive is used to bond a second top layer on to the second top layer or vice versa. The resultant structure is more bulky, less flexible and so less comfortable, and greatly increases the volume of web material required.

The apparatus according to embodiments of the invention in contrast allows sizing of the top sheet sections to overlie the corresponding back sheet sections, minimising any overlap between adjacent top sheet sections, and thus greatly helps to minimize the volume of material required to manufacture the torso encircling portions.

Minimising the amount of material required to manufacture torso encircling portions, by eliminating the need to fold back sheet waist sections, or top sheet waist sections, along their outer edges, and avoiding the need for additional layers of web material, has benefits in terms of reducing material cost and the volume of material that must be disposed of in landfill or other waste disposal facilities after articles incorporating the torso encircling portions have been worn.

The relative arrangement of the components to slit, spread and feed the first and second continuous webs to overlie and entrap the waist and leg elastic strands in discrete regions of the apparatus separates and differentiates the use of welding and adhesive without unnecessarily increasing the numbers of layers of web layers within the resultant torso-encircling portion.

In other embodiments, the apparatus may further include a mechanical bonding mechanism configured to mechanically secure the leg elastic strands in the non-linear pattern between the back sheet leg section and the second top sheet section absent adhesive. Similar benefits flow from the arrangement of the apparatus in that the relative arrangement of the components to slit, spread and feed the first and second continuous webs to overlie and entrap the waist and leg elastic strands in discrete regions of the apparatus allows the ese of welding and mechanical bonding to be separated and differentiated without unnecessarily increasing the numbers of layers of web layers within the resultant torso-encircling portion.

Preferably, so as to improve the comfort and fit of pant-style diapers formed from the resultant torso encircling portions, the apparatus preferably includes a belly elastic infeed apparatus to insert belly elastic strands extending under tension in the third feed direction on to the back sheet leg section, wherein the leg top sheet infeed directs the second top sheet section to overlie the back sheet leg section and sandwich the leg elastic strands and the belly elastic strands therebetween. In such embodiments, the adhesive assembly may be further configured to apply adhesive to one or both of the belly elastic strands and the back sheet leg section to adhere the belly elastic strands between the back sheet leg section and the second top sheet section.

In other embodiments where the apparatus includes a mechanical bonding mechanism, the bonding mechanism may be additionally configured to mechanically secure the leg elastic strands and the belly elastic strands between the back sheet leg section and the second top sheet section absent adhesive.

Preferably, the belly elastic infeed is located to insert belly elastic strands extending under tension in the third feed direction on to the back sheet leg section at the same location as the insertion of the leg elastic strands.

The belly elastic infeed apparatus may include one or more belly elastic feed elements to insert the belly elastic strands to extend in substantially straight lines along the back sheet leg section in the third feed direction. In such embodiments, the belly elastic feed element(s) preferably inserts the belly elastic strands equidistantly spaced from each other in the cross-machine direction to provide a uniform stretch effect.

In embodiments of the invention, the welding assembly may include at least one rotary or blade-style ultrasonic horn with a smooth or grooved working surface and at least one opposing anvil having a working surface defining a plurality of raised ridges, each ridge defining a smooth or grooved working surface. In such embodiments, the horn(s) and anvil(s) are arranged on opposing sides of a feed path through the welding assembly so that the waist elastic strands sandwiched between the first and second back sheet waist sections and the first top sheet section travel in the first feed direction between the horn(s) and anvil(s).

Anchoring welds are formed when the working surfaces of the horn(s) and anvil(s) engage the sheet sections located on opposite sides of the waist elastic strands to melt and at least partially encapsulate the elastic strands and/or frictionally engage the elastic strands in position relative to the facing sheets welds.

In particularly preferred embodiments, the welding assembly includes at least one blade-style ultrasonic horn with a grooved working surface and at least one opposing anvil having a working surface defining a plurality of raised ridges, each ridge defining a smooth contact surface.

The provision of a grooved working surface on the blade-style ultrasonic horn and a plurality of ridges on a working surface of the opposing anvil facilitates the creation of regularly spaced welds and accurate and effective entrapment of the waist elastic strands, which are aligned with the grooves in the working surface of the horn during the welding process.

In such embodiments, the horn(s) and anvil(s) may be configured to form anchoring welds in pairs positioned on opposing sides of a given elastic strand and may partially overlap the given elastic strand. In other embodiments, the welding assembly may be configured to form a single weld or pair of welds that span(s) at least the width of the elastic strand. The anchoring (i.e., entrapment) of the waist elastic strands relative to the back sheet and top sheet sections provides the required elastication in waist sections of the resultant torso encircling portions.

Preferably, each of the plurality of raised ridges is non-linear in shape and the ridges are arranged to define a repeating pattern.

The raised ridges may, for example, include curved or wave-like projections, linear projections, non-linear projections, a plurality of land surfaces and/or notches, a smooth land surface, discrete meandering patterns of raised protrusions (e.g., dots or other geometric shapes), and/or otherwise arranged to create a continuous and repeating pattern on the end product.

In particularly preferred embodiments, the raised ridges may be sinusoidal in shape.

In order to produce a regular series of welds, the repeating pattern of ridges on the working surface of the anvil is positioned to that a peak in each of the ridges is aligned with one of the ultrasonic horns and the juncture between the first and second back sheet waist sections of the first continuous web. Such an arrangement ensures the creation of strong bonds immediately adjacent the second cut line and so ensures the waist elastic strands are secured immediately adjacent the outer edges of waist sections of the eventual diaper product. As a result, a neat finish to the outer edges of the waist sections is achieved once the welded structure is slit along the second cut line.

In other embodiments, in order to achieve a similar effect, the second slitting assembly may be positioned so that the second cut line is aligned with a peak in each of the ridges on the working surface of the anvil(s) and the or one of the ultrasonic horns.

The turnbar assembly preferably includes a first pair of turnbars, each positioned at a first angle to longitudinal and transverse axes of a respective one of the first and second welded strips, and offset relative to each other along the longitudinal direction of travel of the first and second welded strips, so that on wrapping around the first pair of turnbars the first and second welded strips are inverted with one being inverted upstream from the other.

In embodiments of the invention, the first pair of turnbars may be defined by a single roll.

The web guide assembly preferably further includes a second pair of turnbars located immediately upstream or downstream of the first pair of turnbars, each positioned at a second angle relative to longitudinal and transverse axes of a respective one of the first and second welded strips, and offset from each other along the longitudinal direction of travel of the first and second welded strips, so that on wrapping around the second pair of turnbars the first and second welded strips are inverted, with one of the first and second welded strips being inverted upstream from the other. In such embodiments, the first and second angles, and the offset between the turnbars in each of the first and second pairs of turnbars, are selected so that on wrapping around the first and second pairs of turnbars the positions of the second back sheet waist section and the back sheet leg section extending in the third feed direction are swapped relative to the first back sheet waist section in the cross-machine direction such that the back sheet leg section is located between the first and second back sheet waist sections.

It will be appreciated that in embodiments where each turnbar is arranged at an angle of 45° to longitudinal and transverse axes of a welded strip, the longitudinal direction of travel of the welded strip will be rotated through an angle of 90° on wrapping around the turnbar roller.

In embodiments where the turnbar assembly includes first and second pairs of turnbars, the first and second pairs of turnbars may be spaced from each other orthogonally relative to the longitudinal direction of travel of the first and second welded strips between the first and second pairs of turnbars. In such embodiments, the web guide assembly may further include a pair of idler rollers located between the first and second pairs of turnbars and spaced orthogonally from each other relative to the longitudinal direction of travel of the first and second welded strips so that on wrapping around each of the idler rollers each of the first and second welded strips follows a stepped feed path between the first and second pairs of turnbars.

The creation of a stepped feed path between the first and second pairs of turnbars increases the distance of travel between the two pairs of turnbars without increasing the width of the apparatus in a direction generally transverse to the first and third feed directions.

In other embodiments, in order to increase the distance of travel between the two pairs of turnbars without increasing the width of the apparatus in a direction generally transverse to the first and third feed direction, the web guide assembly may include at least one idler roller located between the first and second pairs of turnbars so that on wrapping around the idler roller the first and second welded strips are inverted.

The web guide assembly in other embodiments may include two pairs of idler rollers spaced from each other and angled relative to the first direction of travel so that on passing around a respective one of the idler roller pairs the direction of travel of each of the first and second welded strips is changed and the positions of the first and second welded strips extending in the third feed direction downstream of the web guide assembly are swapped relative to each other in the cross machine direction such that the back sheet leg section is located between the first and second back sheet waist sections.

According to a second aspect of the invention, there is provided a method of manufacturing torso encircling portions for use in the manufacture of pant-style diaper products comprising the steps of:
operating a first web infeed assembly to feed a first continuous web generally in a first feed direction, the first continuous web defining first and second back sheet waist sections and a back sheet leg section extending side by side in the first feed direction with the second back sheet waist section located between the first back sheet waist section and the back sheet leg section;
operating a second web infeed assembly to feed a second continuous web generally in a second feed direction, the second continuous web defining first and second top sheet sections extending side by side in the second feed direction;
operating a first slitting assembly to slit the second continuous web along a first cut line extending in the second feed direction to separate the first and second top sheet sections;
operating a waist elastic infeed to insert waist elastic strands under tension in the first feed direction on to the first and second back sheet waist sections of the first continuous web;
operating a waist top sheet infeed to direct the first top sheet section to overlie the first and second back sheet waist sections and sandwich the waist elastic strands therebetween;
operating a welding assembly to weld the first top sheet section and the first and second back sheet waist sections together intermittently in the first feed direction so as to entrap and secure the waist elastic strands therebetween and form a welded structure;
operating a second slitting assembly to slit the welded structure along a second cut line extending in the first feed direction to separate the welded structure along a juncture between the first and second back sheet waist sections of the first continuous web and thereby define first and second welded strips the first welded strip including the first back sheet waist section, a first portion of the first top sheet section and waist elastic strands secured therebetween, and the second welded strip including the second back sheet waist section, the back sheet leg section, a second portion of the first top sheet section overlying the second back sheet waist section and waist elastic strands secured between the second back sheet waist section and the second portion of the first top sheet section;
operating a web guide assembly to spread the first and second welded strips so that positions of the second back sheet waist section and the back sheet leg section extending in a third feed direction are swapped relative to the first back sheet waist section in a cross-machine direction such that the back sheet leg section is located between the first and second back sheet waist sections;
operating a leg elastic infeed assembly to insert leg elastic strands in a non-linear pattern in the third feed direction on to the back sheet leg section of the first continuous web; and
operating a leg top sheet infeed to direct the second top sheet section to overlie the back sheet leg section and sandwich the leg elastic strands therebetween.

The apparatus and method may be better understood with reference to the following description and accompanying figures, which describe preferred embodiments by way of non-limiting examples. The components in the figures are not necessarily to scale, emphasis instead being placed on illustrating the principles disclosed. Moreover, in the figures, like-reference numerals designate corresponding parts between the different views.
FIG. 1A shows a pant-style diaper product incorporating elasticated torso encircling portions manufactured using apparatus configured in accordance with an embodiment of the invention.
FIGS. 1B and 1C show cross-sectional views of the elasticated torso encircling portions of the pant-style diaper product shown in FIG. 1A.
FIG. 2A is a schematic illustration of the steps of operation of apparatus for manufacturing elasticated torso encircling portions in accordance with an embodiment of the invention.
FIG. 2B is a schematic illustration of the steps of operation of apparatus for manufacturing elasticated torso encircling portions in accordance with another embodiment of the invention.
FIG. 3 is a schematic illustration of apparatus for manufacturing elasticated torso encircling portions in accordance with an embodiment of the invention.
FIGS. 4A and 4B illustrate a web guide assembly suitable for use in the apparatus of FIG. 3 in accordance with the steps of operation illustrated in FIG. 2A.
FIGS. 5A and 5B illustrate a web guide assembly suitable for use in the apparatus of FIG. 3 in accordance with the steps of operation illustrated in FIG. 2B.
FIGS. 6A and 6B illustrate a web guide assembly suitable for use in the apparatus of FIG. 3 in accordance with a further embodiment of the invention.
FIG. 7 illustrates a web guide assembly suitable for use in the apparatus of FIG. 3 in accordance with a yet further embodiment of the invention.
FIG. 8 illustrates a welding assembly suitable for use in the apparatus of FIG. 3 in accordance with an embodiment of the invention.

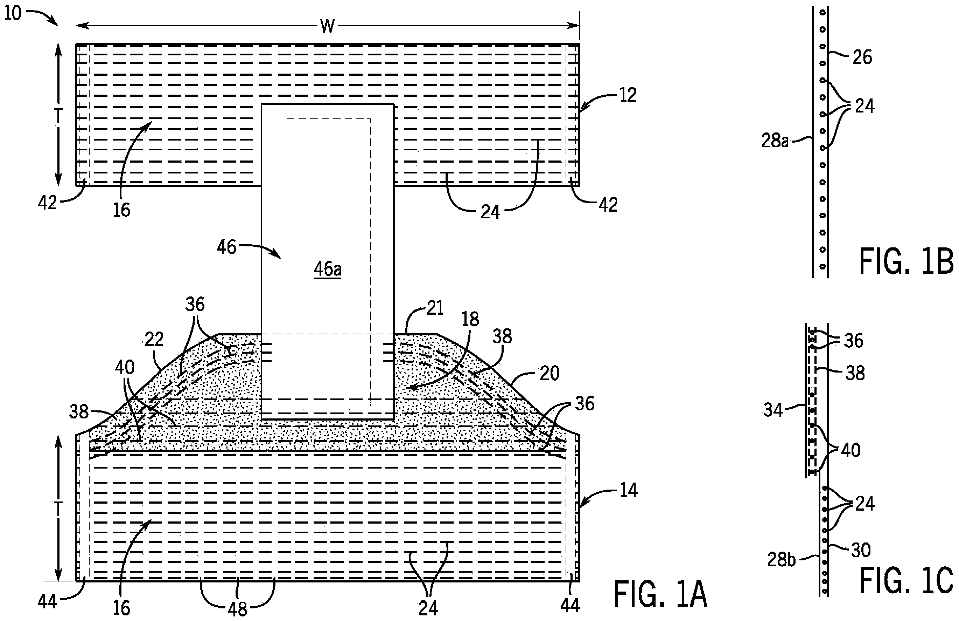

FIGS. 1A-1C show a pant-style diaper product 10 incorporating first and second elasticated torso encircling portions 12,14 manufactured using apparatus configured in accordance with an embodiment of the invention.

The first and second torso encircling portions 12,14 both define a waist region 16. The second torso encircling portion 14 additionally includes a leg region 18 formed integrally with the respective waist region 16, the leg region 18 having curved edges 20,22 separated by a relatively straight edge 21 so as to extend around a wearer's legs when the diaper product 10 is worn. In other embodiments, it is envisaged that the edge 21 extending between the curved edges 20,22 might be shaped. The edge 21 might, for example, be curved.

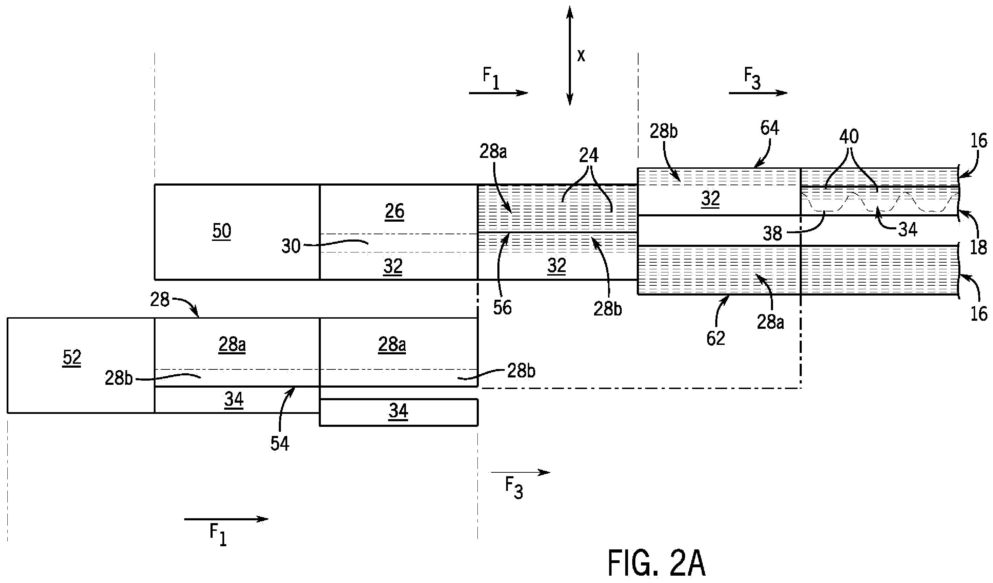

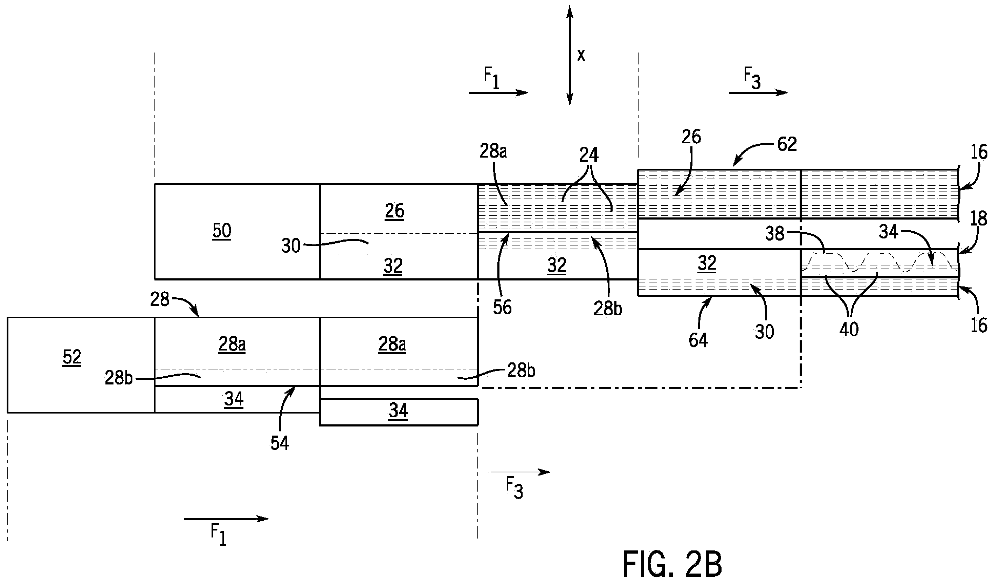

Referring to FIG. 1B, the waist region 16 of the first torso encircling portion 12 includes a plurality of waist elastic strands 24 sandwiched between a first back sheet waist section 26 and a first portion 28a of a first top sheet section 28 (FIGS. 2A and 2B). The waist elastic strands 24 are arranged to extend across all, or substantially all, of the width W of the waist region 16. The waist elastic strands 24 may be equidistantly spaced from each other in a transverse direction T relative to the width W or spaced at varying distances from each other according to design specification.

The first back sheet waist section 26 and the first portion 28a of the first top sheet section 28 are welded to each other on opposite sides of each of the waist elastic strands 24 at intermittent locations along the length of the waist elastic strands 24 so as to anchor or entrap the waist elastic strands 24 in position relative to the first back sheet waist section 26 and the first portion 28a of the first top sheet section 28 at those locations.

Similarly, as shown in FIG. 1C, the waist region 16 of the second torso encircling portion 14 includes a plurality of waist elastic strands 24 sandwiched between a second back sheet waist section 30 and a second portion 28b of the first top sheet section 28 (FIGS. 2A and 2B). As with the waist region 16 of the first torso encircling portion 12, the waist elastic strands 24 are arranged to extend across all or substantially all of the width W of the waist region 16 and may be equidistantly spaced from each other or spaced at varying distances in a transverse direction T relative to the width W.

The second back sheet waist section 30 and the second portion 28b of the first top sheet section 28 are welded to each other on opposite sides of each of the waist elastic strands 24 at intermittent locations along the length of the waist elastic strands 24 so as to anchor or entrap the waist elastic strands 24 in position relative to the second back sheet waist section 30 and the second portion 28b of the first top sheet section 28 at those locations.

The leg region 18 of the second torso encircling portion 12 includes a plurality of leg elastic strands 36 sandwiched between a back sheet leg section 32 (FIGS. 2A and 2B) and a second top sheet section 34, the leg elastic strands 36 being arranged in one exemplary embodiment to follow the outline of the curved edges 20,22 of the leg region 18. The leg elastic strands 36 are bonded in place by means of adhesive 38 provided between the back sheet leg section 32 and the second top sheet section 34.

In the embodiment shown in FIGS. 1A-1C, a plurality of belly elastic strands 40 are also sandwiched between the back sheet leg section 32 (FIGS. 2A and 2B) and the second top sheet section 34 by means of adhesive 38. The belly elastic strands 40 are arranged to extend across all or substantially all of the width W of the leg region 18 and may be equidistantly spaced from each other in a transverse direction T relative to the width W of spaced apart at varying intervals. In other embodiments, the belly elastic strands 40 may be omitted.

The use of welding to anchor or entrap the waist elastic strands 24 between the respective back sheet waist sections 26,30 and the respective portions 28a,28b of the first top sheet section 28 ensures a smooth finished edge and thus eliminates the need to fold the back sheet waist sections along their outer edges to secure the outermost edge of the resultant waist portions.

While the product 10 is shown in FIG. 1A in an open format for clarity, it will be understood that the first torso encircling portion 12 and the second torso encircling portion 14 are joined together at side seam regions 42,44 in the completed product 10. Side seam regions 42,44 may be secured by means of adhesive of ultrasonic welds.

In use, the first and second portions 28a,28b of the first top sheet section 28 and the second top sheet section 34 between them define a skin contact surface of the diaper product 10 whilst the first and second back sheet waist sections 26,30 and the back sheet leg section 32 between then define an outer surface of the diaper product 10. The back and top sheet sections 26,28,30,32,34 are preferably formed from nonwoven materials.

As shown in FIG. 1A, the first and second torso encircling portions 12,14 are bridged by a crotch portion 46 coupled at one end to the waist region 16 of the first torso encircling portion 12 and at its other end to the leg region 18 of the second torso encircling portion 14. The crotch portion 46 includes a liquid absorbent layer on an upper surface 46a and an outer, liquid impermeable layer on its underside (not shown).

The elasticity provided by the waist elastic strands 24 and belly elastic strands 40 may be deactivated (i.e., cut or otherwise treated to reduce the elasticity therein) in these regions so as to prevent bunching of the crotch portion 46.

In other embodiments, it is envisaged that the leg elastic strands 36 may be mechanically secured in place between the back sheet leg section 32 and the second top sheet section 34 absent adhesive.

In such embodiments, the belly elastic strands 40 may similarly be mechanically secured between the back sheet leg section 32 and the second top sheet section 34 absent adhesive.

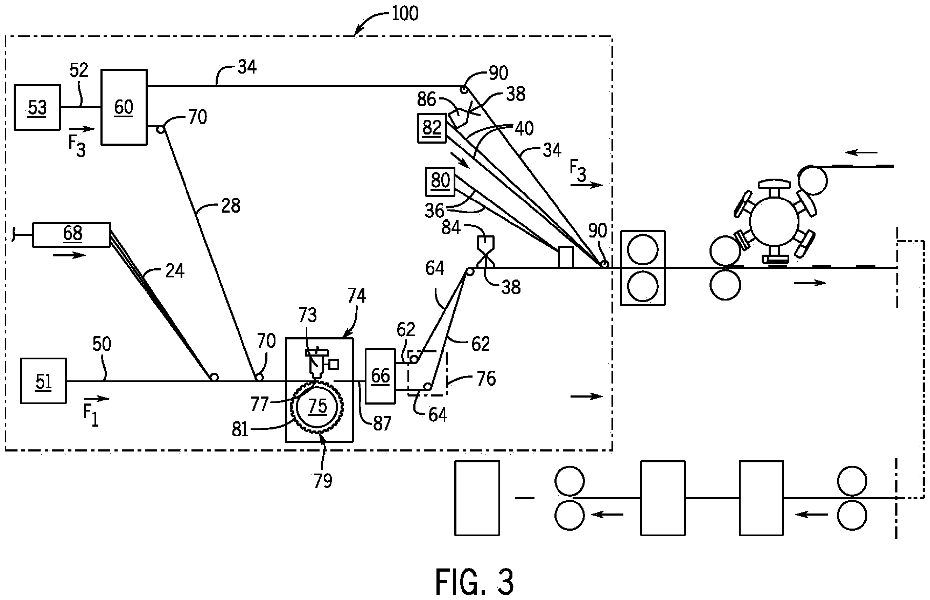

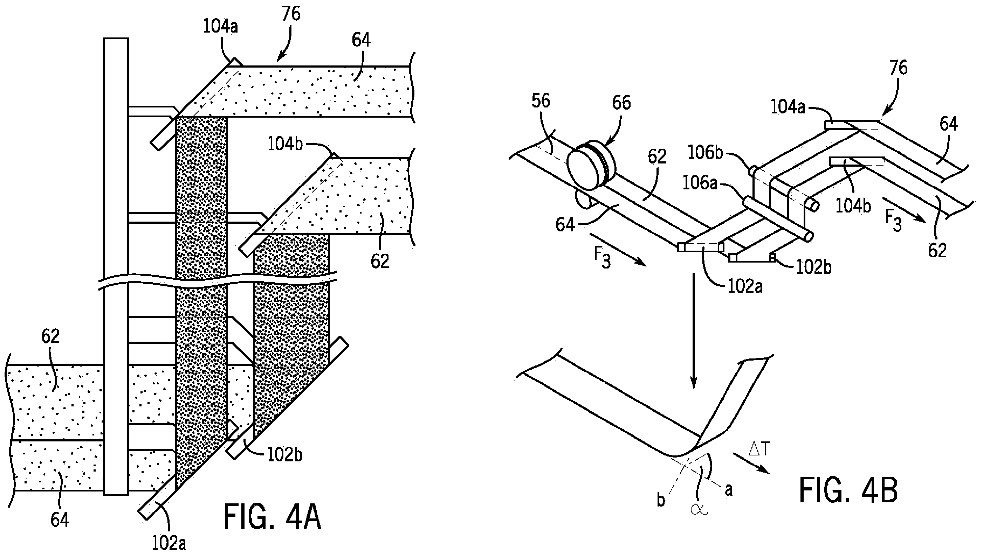

A method and apparatus for forming the first and second torso encircling portions 12,14 will now be described with reference to FIG. 2A and the apparatus shown in FIGS. 3, 4A and 4B.

As a first step, a first continuous web 50 of nonwoven material is fed generally in a first feed direction F₁ by means of a first web infeed assembly 51, and a second continuous web 52 of nonwoven material is fed generally in a second feed direction F₂ by means of a second web infeed assembly 53.

The first continuous web 50 defines the first and second back sheet waist sections 26,30 and the back sheet leg section 32 extending side by side in the first feed direction F₁ with the second back sheet waist section 30 located between the first back sheet waist section 26 and the back sheet leg section 32.

The second continuous web 52 defines the first top sheet section 28 and the second top sheet section 34 extending in the second feed direction F₂. It will be appreciated that in order to avoid excess material wastage, the first top sheet section 28 may correspond substantially in width in a cross-machine direction X to the width of the first and second back sheet waist sections 26,30 in the cross-machine direction X. Similarly, the second top sheet section 34 preferably corresponds substantially in width in a cross-machine direction X to the width of the back sheet leg section 32 in the cross-machine direction X.

The second continuous web 52 is slit by means of a first slitting assembly 60 including one or more knives, blades or other cutting devices along a first cut line 54 extending in the second feed direction Fz to form the first and second top sheet sections 28,34.

The first slitting assembly 60 may also include one or more web spreaders, which act to direct the first and second top sheet sections 28,34 downstream from the first slitting and spreading assembly 60 so that the first and second top sheet sections 28,34 are be arranged adjacent to, but spaced from, each other in a cross-machine direction X.

A waist elastic infeed in the form of a harp 68 inserts waist elastic strands 24 extending in the first feed direction F₁ under tension to overlay the first and second back sheet waist sections 26,30. The harp 68 feeds the waist elastic strands 24 in substantially straight lines in the first feed direction F₁ along the first and second back sheet waist sections 26,30. The waist elastic strands 24 may be equidistantly spaced in a direction T transverse to the first feed direction F₁ or be fed at a spacing that varies in the transverse direction T between adjacent waist elastic strands 24 according to product design specifications.

The first top sheet section 28 is directed via a waist top sheet infeed 70 to overlay the first and second back sheet waist sections 26,30 and sandwich the waist elastic strands 24 between the first top sheet section 28 and the first and second back sheet waist sections 26,30.

In order to secure the first and second back sheet waist sections 26,30 and the first top sheet section 28 together, and retain the waist elastic strands 24 in position, the first and second back sheet waist sections 26,30 and the first top sheet section 28 are fed through at least one welding assembly 74.

The at least one welding assembly 74 may be any known mechanical bonding system including, as non-limiting examples, a thermal welding system, a pressure welding system, a rotary ultrasonic welding system, or a blade ultrasonic welding system, any of which includes one or more components that cooperate to weld (i.e. fuse) the first and second back sheet sections 26,30 and the first top sheet section 28 together.

In one embodiment, the welding assembly 74 is provided in the form of an ultrasonic bonding system having one or more blade-style horns 73, also known as a sonotrode, with a grooved working surface 77 that cooperate with one or more opposing anvils 75 with a smooth or ridged working surface 79. In other embodiments, the welding assembly 74 may be provided in the form of an ultrasonic bonding system including one or more rotary or blade-style horn(s) having either a smooth or grooved working surface and one or more anvils with grooved ridges.

While welding assembly 74 and corresponding horn and anvil elements 73 and 75 are referred to in the singular tense below, it is contemplated that any of the described embodiments may include multiple welding assemblies, each including one or more horn and/or anvil components.

The first and second back sheet waist sections 26,30 and the first top sheet section 28 are fed through the welding assembly 74 so that the waist elastic strands 24 under tension in the first feed direction F₁ are aligned with the grooves formed in the working surface of the ultrasonic horn. Intermittent operation of the ultrasonic horn 73 forms welds 48 at intermittent locations along the length of the waist elastic strands 24 as the first and second back sheet waist sections 26,30 and the first top sheet section 28 and waist elastic strands 24 are fed through the welding assembly 74.

Operation of the ultrasonic horn 73 melts the nonwoven material of the first and second back sheet waist sections 26,30 and the first top sheet section 28 at the points of contact between the horn 73 and the anvil 75, which results in the formation of welds 48 between the respective back sheet and top sheet sections 26,30,28, the welds 48 being aligned with land surfaces between the grooves in the working surface 77 of the ultrasonic horn 73 so that they entrap the waist elastic strands 24.

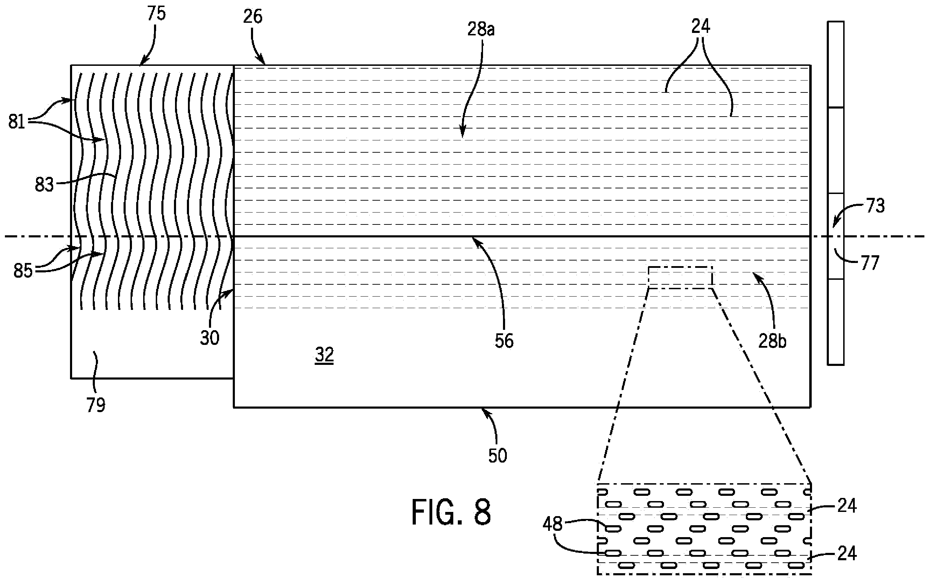

A particularly preferred ultrasonic welding system 74 is illustrated schematically in FIG. 8 and includes a plurality of blade-style horns 73, or sonotrodes. Each blade-style horn 73 includes a grooved working surface 77 that cooperates with an opposing anvil 75 having a ridged working surface 79. As shown in FIG. 8, the working surface 79 includes a plurality of raised ridges 81 equidistantly spaced so as to form a repeating pattern and presenting smooth contact surfaces 83 for contact with the grooved working surface 77 of the opposing ultrasonic horns 73. Each of the ridges 81 is sinusoidal in shape defining periodic peaks 85 along its length.

As shown schematically in FIG. 8, the working surface 79 of the anvil 75 is preferably positioned so that a series of peaks 85 of adjacent ridges 81 are aligned with an ultrasonic horn 73 and a second slit line 56, which is aligned with a juncture between the first and second back sheet waist sections 26,30.

The resultant welded structure 87, resulting from welding of the first and second back sheet waist sections 26,30 and the first top sheet section 28 to entrap the waist elastic strands 24, is slit by means of a second slitting assembly 66 including one or more knives, blades, or other cutting devices along a second cut line 56 extending in the first feed direction F₁ to create first and second welded strips 62,64. The second cut line 56 separates the welded structure along a juncture between the first and second back sheet waist sections 26,30. As a result, the first welded strip 62 includes the first back sheet waist section 26, the first portion 28a of the first top sheet section 28, overlying the first back sheet waist section 26, and waist elastic strands 24 secured therebetween. The second welded strip 64 includes the second back sheet waist section 30, the second portion 28b of the first top sheet section 28, overlying the second back sheet waist section 30, and waist elastic strands 24 therebetween.

In embodiments employing the ultrasonic horn 73 and anvil 75 arrangement shown in FIG. 8, the alignment of the peaks 85 of the ridges 81 on the working surface 79 of the anvil 75 ensures the creation of strong bonds immediately adjacent the second cut line 56. As a result it ensures the waist elastic strands 24 are secured immediately adjacent the slit edges of the first and second welded strips 62,64. This results in a neat finish along the slit edges of the first and second welded strips 62,64 and eliminates, or at least minimises, the need for additional finishing of those edges.

It will be appreciated that a similar effect may be achieved in other embodiments by aligning the second slitting assembly 62 so that the second cut line 56 is aligned with peaks 85 of the ridges 81 on the working surface 79 of the anvil 75 and the ultrasonic horn 73.

Following slitting of the welded structure 87, the resultant first and second welded strips 62,64 are fed through at least one web guide assembly 76 arranged to spread the first and second welded strips 62,64 so that positions of the second back sheet waist section 30 and the first back sheet waist section 26 extending in a third feed direction F₃ are swapped relative to the first back sheet waist section 26 in the cross-machine direction X such that. the back sheet leg section 32 is located between the first and second back sheet waist sections 26,30, as illustrated schematically in FIG. 2A.

Referring to FIGS. 4A and 4B, the web guide assembly 76 includes a first pair of turnbars 102a,102b, each positioned at a first angle α relative to longitudinal and transverse axes a,b of a respective one of the first and second welded strips 62,64. The first pair of turnbars 102a,102b are also offset relative to each other along the longitudinal direction of travel DT of the first and second welded strips 62,64 so that on wrapping around the first pair of turnbars 102a,102b, the first and second welded strips 62,64 are inverted, with one being inverted upstream from the other.

In the embodiment shown in FIGS. 4A and 4B, each of the first pair of turnbars 102a,102b is positioned at an angle α of 45° relative to longitudinal and transverse axes a,b of each of the first and second welded strips 62,64 such that the longitudinal direction of travel DT of each of the first and second welded strips 62,64 is rotated through a lefthand angle of 90° on passing around the first pair of turnbars 102a,102b. It will be appreciated that the angle α may be varied in order to define a preferred feed path through the turnbar web guide assembly 76, which may be dictated by the space available within the apparatus 100.

In addition, the first pair of turnbars 102a,102b are offset relative to each other along the longitudinal direction of travel of the first and second welded strips 62,64 so that rotation through a lefthand angle of 90° in the direction of travel DT of the second welded strip 64 occurs upstream from rotation through a lefthand angle of 90° in the direction of travel DT of the first welded strip 62.

In the embodiment shown in FIGS. 4A and 4B, the web guide assembly 76 further includes a second pair of turnbars 104a,104b located downstream of the first pair of turnbars 102a,102b. Each of the second pair of turnbars 104a,104b is positioned at a second angle relative to the longitudinal and transverse axes of a respective one of the first and second welded strips 62,64 such that on passing around a respective one of the second pair of turnbars 104a,104b, the first and second welded strips 62,64 are again inverted.

In addition, each of the second pair of turnbars 104a,104b is positioned at an angle α of 135° relative to the longitudinal and transverse axes a,b of each of the first and second welded strips 62,64.

As a result, on passing around the second pair of turnbars 104a,104b, the longitudinal direction of travel DT of each of the first and second welded strips 62,64 is rotated through a righthand angle of 90° such that the third feed direction F₃ downstream of the second pair of turnbars 104a,104b is essentially parallel to the first feed direction F₁ upstream from the web guide assembly 76.

In addition, the second pair of turnbars 104a,104b are offset relative to each other along the longitudinal direction of travel of the first and second welded strips 62,64 so that rotation through a righthand angle of 90° in the direction of travel DT of the first welded strip 62 occurs upstream from rotation through a righthand angle of 90° in the direction of travel DT of the second welded strip 64.

The net result of the first and second angles of the first and second pairs of turnbars 102a,102b,104a,104b, and the offset between the respective turnbars in each pair of turnbars, results in the positions of the second back sheet waist section 30 and the back sheet leg section 32 extending in the third feed direction F₃ being swapped relative to the first back sheet waist section 26 in the cross-machine direction such that the back sheet leg section 32 is located between the first and second back sheet waist sections 26,30, as shown schematically in FIG. 2A.

It will be appreciated that in other embodiments, the first and second angles of the first and second pairs of turnbars 102a,102b,104a,104b might be varied depending on the setup of manufacturing processes upstream and downstream from the web guide assembly 76 and/or the space available for positioning the individual components of the web guide assembly 76. The only limiting factor is that the turnbars cause the positions of the second back sheet waist section 30 and the back sheet leg section 32 extending in a third feed direction to be swapped relative to the first back sheet waist section 26 in a cross-machine direction such that the back sheet leg section 32 is located between the first and second back sheet waist sections 26,30. For example, the first pair of turnbars 102a,102b might be angled relative to the longitudinal and transverse axes a,b of each of the first and second welded strips 62,64 so that the direction of travel DT of each of the first and second welded strips 62,64 is rotated through a righthand angle of 90° on passing around the first pair of turnbars 102a,102b, or any other angle, as required. Likewise, the second pair of turnbars 104a,104b might be angled so each of the first and second welded strips 62,64 is rotated through a lefthand angle of 90°, or any other angle, as required.

The web guide assembly 76 shown in FIG. 4B includes a pair of idler rollers 106a,106b located between the first pair of turnbars 102a,102b and the second pair of turnbars 104a,104b. The idler rollers 106a,106b are spaced from each other orthogonally relative to the longitudinal direction of travel of the first and second welded strips 62,64 so that, on wrapping around a respective one of the idler rollers 106a,106b, each of the first and second welded strips 62,64 follows a stepped path as it is fed from the first pair of turnbars 102a,102b towards the second pair of turnbars 104a,104b. The creation of a stepped path between the first and second pairs of turnbars 102a,102b,104a,104b is advantageous is circumstances where the next step of the manufacturing process, i.e. the step occurring immediately downstream of the second pair of turnbars 104a,104b, occurs in a different plane to that of the welding assembly 74 upstream from the first pair of turnbars 102a,102b.

It is envisaged that in other embodiments, each idler roller 106a,106b may be provided in the form of a pair of rollers so that each of the welded strips 62,64 is wrapped around a separate roller.

In further embodiments, where, for example, it is not necessary to change the plane of the webpath of the first and second welded strips 62,64 - i.e. it is not necessary to create a step in the webpath of the first and second welded strips 62,64 between the first and the second pairs of turnbars 102a,102b,104a,104b - the idler rollers 106a,106b might be omitted.

A leg elastic infeed assembly in the form of a curved leg elastic laydown unit 80 is located downstream from the web guide assembly 76 to insert leg elastic strands 36 extending under tension generally in the third feed direction F₃ - i.e. in the first feed direction F₁ - on to the back sheet leg section 32 of the first continuous web 50 in a periodic wave pattern or other non-linear pattern along the back sheet leg section 32 in the third feed direction F₃. For example, the curved leg elastic laydown unit 80 may be provided as one or more swinging arms that can be include eyelets to receive individual strands of the leg elastic strands 36. The swinging arms can be programmed to move in a predetermined pattern (e.g., oscillating from side to side), and the eyelets can pull the leg elastic strands 36 according to the motion of the swinging arms. In this way, the curved leg elastic laydown unit 80 can adjust the curvature of the leg elastic strands 36 while applying the same to the back sheet leg section 32. The swinging arms can also be maintained in a constant position to apply the leg elastic strands 36 in a substantially straight configuration for all or select portions of the length of travel, if desired.

It will be appreciated that the curved leg elastic laydown unit 80 may be controlled so as to vary the shape defined by the leg elastic strands 36 on the back sheet leg section 32 depending on the intended end use of the torso encircling portions 12,14.

An adhesive assembly including a first adhesive applicator 84 is configured to apply adhesive 38 to the back sheet leg section 32 immediately upstream from the insertion of the leg elastic strands 36 so that the leg elastic strands 36 laid down by the curved leg elastic laydown unit 80 adhere to the back sheet leg section 32 adjacent the waist elastic strands 24 secured between the second back sheet waist section 30 and the second portion 28b of the first top sheet section 28.

It will be appreciated that in other embodiments, the first adhesive applicator 84 might be configured to apply adhesive 38 directly to the leg elastic strands 36 as they are laid down by the curved leg elastic laydown unit 80 to adhere to the back sheet leg section 32.

A belly elastic infeed in the form of a belly elastic laydown unit 82 may be located downstream or upstream of the curved leg elastic laydown unit 80 to insert belly elastic strands 40 extending under tension in the third feed direction F₃ on to the adhesive on the back sheet leg section 32. Preferably, however, the belly elastic infeed in the form of the belly elastic laydown unit 82 is located so as to insert belly elastic strands extending under tension in the third feed direction F₃ on to the back sheet leg section 32 at the same location as the insertion of the leg elastic strands 36 by means of the curved leg elastic laydown unit 80.

The belly elastic laydown unit 82 feeds the belly elastic strands 40 in substantially straight lines in the third feed direction F₃ along the back sheet leg section 32, equidistantly spaced in a direction T transverse to the third feed direction F₃. In other embodiments, the belly elastic strands 40 and associated belly elastic laydown unit 82 may be omitted.

The belly elastic strands 40 may be equidistantly spaced in a direction T transverse to the first feed direction F₁ or be fed at a spacing that varies in the transverse direction T between adjacent belly elastic strands 40 according to product design specifications.

The second top sheet section 34 is then directed via a leg top sheet infeed 90 to overlay the back sheet leg section 32 so as to sandwich leg elastic strands 36 and belly elastic strands 40 therebetween, the second top sheet section 34 being bonded to the back sheet leg section 32 by means of the adhesive 38.

In the embodiment illustrated in FIG. 3, the adhesive assembly includes a second adhesive applicator 86 configured to apply adhesive 38 to the second top sheet section 34 as it is fed to overlay the back sheet leg section 32.

In other embodiments, the second adhesive applicator 86 may be omitted and the second top sheet section 34 may be adhered to the back sheet leg section 32 by means of the adhesive 38 applied by the first adhesive applicator 84.

It will be appreciated that in other embodiments each adhesive applicator 84,86 may be configured to apply adhesive 38 directly to the leg elastic strands 36 and/or any belly elastic strands 40.

It will also be appreciated that in other embodiments the use of adhesive might be omitted altogether and a mechanical bonding mechanism may be provided to mechanically secure the leg elastic strands 36 and any belly elastic strands 40 between the back sheet leg section 32 and the second top sheet section 34 absent adhesive.

It will also be appreciated that other web guide assemblies 76 might be utilised in the apparatus 100 in order to achieve the required swapping of the position of the first back sheet waist section 26 in a cross-machine direction X such that the back sheet leg section 32 is located between the first and second back sheet waist sections 26,30.

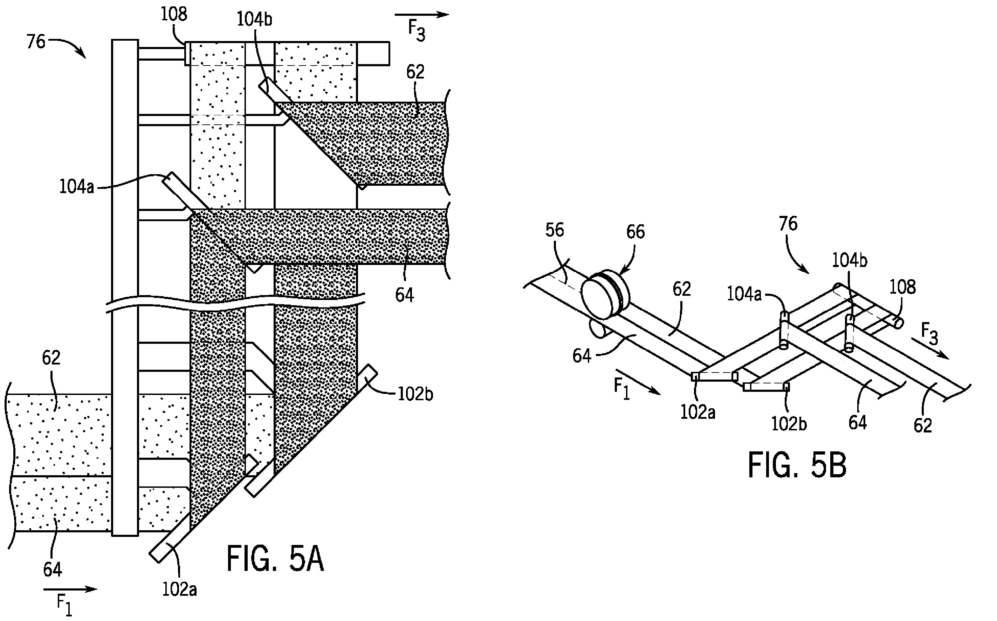

One such embodiment of web guide assembly 76 will be described with reference to FIGS. 2B, 5A and 5B.

In this embodiment, and as shown in FIGS. 5A and 5B, the web guide assembly 76 includes a first pair of turnbars 102a,102b, each positioned at a first angle α relative to the longitudinal and transverse axes a,b of the first and second welded strips 62,64. The first pair of turnbars 102a,102b are offset relative to each other along the longitudinal direction of travel DT of the first and second welded strips 62,64 so that on wrapping around the first pair of turnbars 102a,102b, the first and second welded strips 62,64 are inverted, with one being inverted upstream from the other.

Each of the first pair of turnbars 102a,102b is positioned at an angle α of 45° relative to longitudinal and transverse axes a,b of each of the first and second welded strips 62,64 such that the longitudinal direction of travel DT of each of the first and second welded strips 62,64 is rotated through a lefthand angle of 90° on passing around the first pair of turnbars 102a,102b.

The first pair of turnbars 102a,102b are also offset relative to each other along the longitudinal direction of travel of the first and second welded strips 62,64 so that rotation through a lefthand angle of 90° in the direction of travel DT of the second welded strip 64 occurs upstream from rotation through a lefthand angle of 90° in the direction of travel DT of the first welded strip 62.

The web guide assembly 76 shown in FIGS. 5A and 5B further includes an idler roller 108 downstream from the first pair of turnbars 102a,102b, which is positioned perpendicular to the longitudinal and transverse axes a,b of each of the first and second welded strips 62,64. As a result, on wrapping around the idler roller 108, the direction of travel of the first and second welded strips 62,64 is reversed and the welded strips are re-inverted.

The first and second welded strips 62,64 are then fed to a second pair of turnbars 104a,104b positioned at an angle α of 45° relative to longitudinal and transverse axes a,b of each of the first and second welded strips 62,64. As a result, the longitudinal direction of travel DT of each of the first and second welded strips 62,64 is again rotated through a lefthand angle of 90° on passing around the second pair of turnbars 104a,104b.

The inclusion of the idler roller 108 not only facilitates an additional inversion of the first and second welded strips 62,64 but also extends the feed path between the first and second pairs of turnbars 102,102b,104a,104b without unduly increasing the cross-machine dimension of the apparatus.

As with the first pair of turnbars 102a,102b, the welded strips 62,64 are again inverted on passing around the second pair of turnbars 104a,104b. The second pair of turnbars 104a,104b are, however, offset relative to each other along the longitudinal direction of travel of the first and second welded strips 62,64 so that the rotation through a lefthand angle of 90° in the direction of travel DT of the first welded strip 62 occurs upstream through a lefthand angle of 90° in the direction of travel DT of the second welded strip 64. As a result, whilst the first and second welded strips 62,64 are inverted when compared with the orientation of the first and second welded strips 62,64 immediately upstream from the web guide assembly 76, the positions of the second back sheet waist section 30 and the back sheet leg section 32 extending in the third feed direction F₃ being swapped relative to the first back sheet waist section 26 in the cross-machine direction such that the back sheet leg section 32 is located between the first and second back sheet waist sections 26,30, as shown schematically in FIG. 2B.

Thereafter, the curved leg elastic laydown unit 80 inserts leg elastic strands 36 on to the inverted back sheet leg section 32 in the same manner as described above with reference to FIGS. 2A, 3, 4A and 4B. The belly elastic laydown unit 82, if used, similarly inserts belly elastic strands 40 on to the inverted back sheet leg section 32, and the leg top sheet infeed 90 directs the second top sheet section 34 to overlay the inverted back sheet leg section 32 in the same manner as previously described with reference to FIGS. 2A, 3, 4A and 4B. Accordingly, we will not repeat the description of those steps of operation of the apparatus 100, which are otherwise identical save for the inversion of the back sheet leg section 32 in the embodiment illustrated schematically in FIGS. 2B, 5A and 5B.

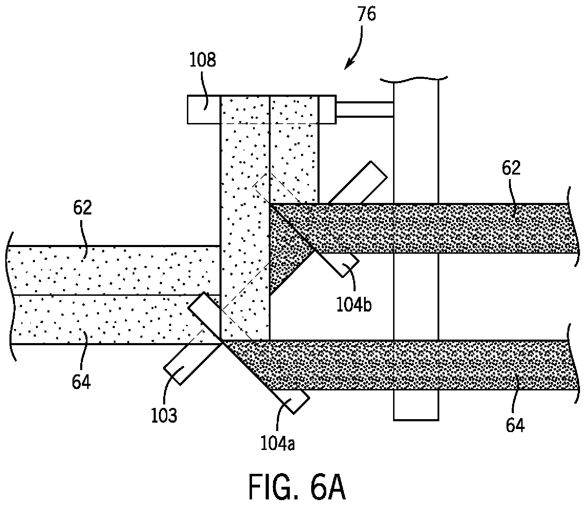

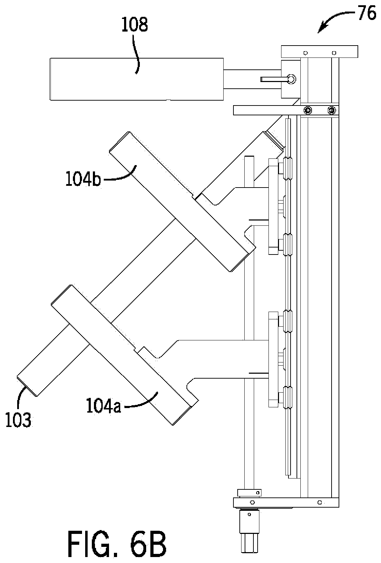

Another embodiment of web guide 76 may be described by reference to FIGS. 2B, 6A and 6B.

In this embodiment, the web guide assembly 76 includes a first pair of turnbars in the form of a single turnbar 103 positioned at a first angle angle α relative to the longitudinal and transverse axes a,b of the first and second welded strips 62,64. As a result, the first and second welded strips 62,64 wrap around the turnbar 103 at offset locations such that the first and second welded strips 62,64 are inverted, with one being inverted upstream from the other.

Referring to FIG. 6A, it can be seen that the turnbar 103 is positioned at an angle α of 45° relative to longitudinal and transverse axes a,b of each of the first and second welded strips 62,64 such that the longitudinal direction of travel DT of each of the first and second welded strips 62,64 is rotated through a lefthand angle of 90° on passing around the turnbar 103.

The relative positioning of the turnbar 103 relative to the longitudinal axes a,b of each of the first and second welded strips 62,64 means that the rotation through a lefthand angle of 90° in the direction of travel DT of the second welded strip 64 occurs upstream from the rotation through a lefthand angle of 90° in the direction of travel DT of the first welded strip 62.

The web guide assembly 76 shown in FIGS. 6A and 6B further includes an idler roller 108 downstream from the turnbar 103, which is positioned perpendicular to the longitudinal and transverse axes a,b of each of the first and second welded strips 62,64. As a result, on wrapping around the idler roller 108, the direction of travel of the first and second welded strips 62,64 is reversed and the welded strips are re-inverted.

The first and second welded strips 62,64 are then fed to a second pair of turnbars 104a,104b positioned at an angle α of 45° relative to longitudinal and transverse axes a,b of each of the first and second welded strips 62,64. As a result, the longitudinal direction of travel DT of each of the first and second welded strips 62,64 is again rotated through a lefthand angle of 90° on passing around the second pair of turnbars 104a,104b.

As with turnbar 103, the welded strips 62,64 are again inverted on passing around the second pair of turnbars 104a,104b. The second pair of turnbars 104a,104b are, however, offset relative to each other along the longitudinal direction of travel of the first and second welded strips 62,64 so that the rotation through a lefthand angle of 90° in the direction of travel DT of the first welded strip 62 occurs upstream through a lefthand angle of 90° in the direction of travel DT of the second welded strip 64. As a result, whilst the first and second welded strips 62,64 are inverted when compared with the orientation of the first and second welded strips 62,64 immediately upstream from the web guide assembly 76, the positions of the second back sheet waist section 30 and the back sheet leg section 32 extending in the third feed direction F₃ being swapped relative to the first back sheet waist section 26 in the cross-machine direction such that the back sheet leg section 32 is located between the first and second back sheet waist sections 26,30, as shown schematically in FIG. 2B.

Again, as with the embodiment described above with reference to FIGS. 2B, 5A and 5B, thereafter the curved leg elastic laydown unit 80 inserts leg elastic strands 36 on to the inverted back sheet leg section 32 in the same manner as described above with reference to FIGS. 2A, 3, 4A and 4B. The belly elastic laydown unit 82 similarly inserts belly elastic strands 40 on to the inverted back sheet leg section 32, and the leg top sheet infeed 90 directs the second top sheet section 34 to overlay the inverted back sheet leg section 32 in the same manner as previously described with reference to FIGS. 2A, 3, 4A and 4B. Accordingly, we will not repeat the description of those steps of operation of the apparatus 100, which are otherwise identical save for the inversion of the back sheet leg section 32 in the embodiment illustrated schematically in FIGS. 2B, 6A and 6B.

It will be appreciated that the resultant effect of the inversion of the back sheet leg section 32 in the embodiment shown and described with reference to FIGS. 2B, 5A and 5B, as well as the embodiment shown and described with reference to FIGS. 2B, 6A and 6B, is that the leg elastic strands 36 and the belly elastic strands 40 are sandwiched between the second top sheet section 34 on an opposite side of the substrate forming the second back sheet waist section 30 and the back sheet leg section 32 to the waist elastic strands 24 sandwiched between the second portion 28b of the first top sheet section 28 and the second back sheet waist section 30. It remains the case, however, that the apparatus and method described with reference to FIGS. 2B, 5A and 5B, as well as the apparatus and method described with reference to FIGS. 2B, 6A and 6B, achieves the same benefits in terms of minimising any overlap between adjacent top sheet sections as the apparatus and method described with reference to FIGS. 2A, 4A and 4B. It remains the case that the embodiments described with reference to FIGS. 2B, 5A, 5B, 6A and 6B greatly helps to minimize the volume of material required to manufacture the torso encircling portions.

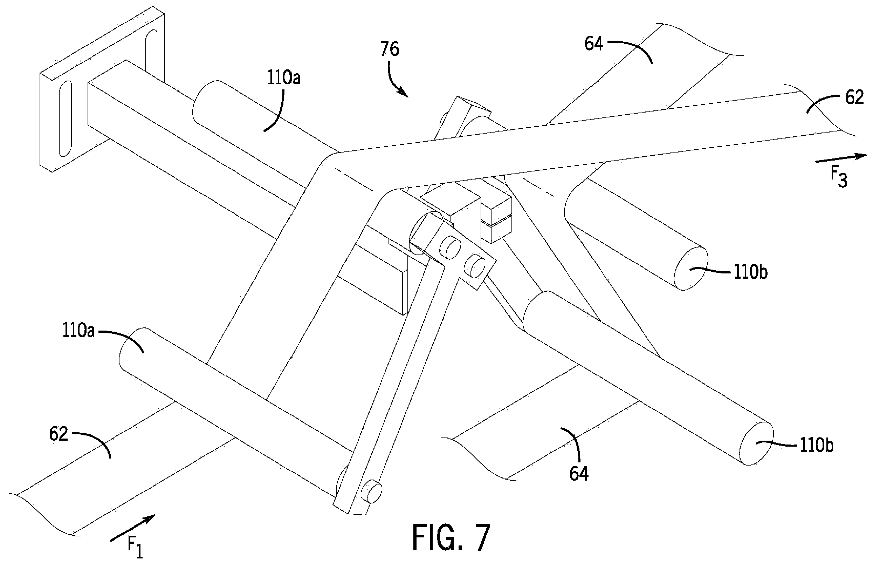

One further embodiment of web guide 76 may be described by reference to FIGS. 2A and 7.

In this embodiment, the web guide assembly 76 includes two pairs of idler rollers 110a,110b spaced from each other and angled relative to the first direction of travel F₁ so that on passing around a respective pair of idler rollers 110a,110b, the direction of travel DT of each of the first and second welded strips 62,64 is changed.

As illustrated in FIG. 7, by selecting the relative positions and angular orientation of the idler rollers 110a,110b it is possible to change the direction of travel DT of each of the first and second welded strips 62,64 such that the positions of the first and second welded strips 62,64 extending in the third feed direction F₃ downstream of the web guide assembly 76 are swapped relative to each other in the cross-machine direction X.

As a result, the positions of the second back sheet waist section 30 and the back sheet leg section 32 extending in the third feed direction F₃ are swapped relative to the first back sheet waist section 26 in the cross-machine direction such that the back sheet leg section 32 is located between the first and second back sheet waist sections 26,30, as shown schematically in FIG. 2A.

Again, as with the embodiment described above with reference to FIGS. 2B, 5A and 5B, thereafter the curved leg elastic laydown unit 80 inserts leg elastic strands 36 on to the back sheet leg section 32 in the same manner as described above with reference to FIGS. 2A, 3, 4A and 4B. The belly elastic laydown unit 82 similarly inserts belly elastic strands 40 on to the inverted back sheet leg section 32, and the leg top sheet infeed 90 directs the second top sheet section 34 to overlay the inverted back sheet leg section 32 in the same manner as previously described with reference to FIGS. 2A, 3, 4A and 4B. Accordingly, we will not repeat the description of those steps of operation of the apparatus 100, which are otherwise identical.

Embodiments of the invention will now be described in the following paragraphs:
1. Apparatus (100) for manufacturing torso encircling portions (12,14) of a pant-style diaper product, the apparatus (100) comprising:
   a first web infeed assembly (51) to feed a first continuous web (50) generally in a first feed direction (F₁), the first continuous web (50) defining first and second back sheet waist sections (26,30) and a back sheet leg section (32) extending side by side in the first feed direction (F₁) with the second back sheet waist section (30) located between the first back sheet waist section (26) and the back sheet leg section (32);
   a second web infeed assembly (53) to feed a second continuous web (52) generally in a second feed direction (Fz), the second continuous web (52) defining first and second top sheet sections (28,34) extending side by side in the second feed direction (Fz);
   a first slitting assembly (60) configured to slit the second continuous web (52) along a first cut line (54) extending in the second feed direction (F₂) to separate the first and second top sheet sections (28,34);
   a waist elastic infeed (68) configured to insert waist elastic strands (24) under tension in the first feed direction (F₁) on to the first and second back sheet waist sections (26,30) of the first continuous web (50);
   a waist top sheet infeed (70) configured to direct the first top sheet section (28) to overlie the first and second back sheet waist sections (26,30) and sandwich the waist elastic strands (24) therebetween;
   a welding assembly (74) configured to weld the first top sheet section (28) and the first and second back sheet waist sections (26,30) together intermittently in the first feed direction (F₁) so as to entrap and secure the waist elastic strands (24) therebetween and form a welded structure (83);
   a second slitting assembly (62) configured to slit the welded structure (83) along a second cut line (56) extending in the first feed direction (F₁) to separate the welded structure (83) along a juncture between the first and second back sheet waist sections (26,30) of the first continuous web (50) and thereby define first and second welded strips (62,64), the first welded strip (62) including the first back sheet waist section (26), a first portion (28a) of the first top sheet section (28) and waist elastic strands (24) secured therebetween, and the second welded strip (64) including the second back sheet waist section (30), the back sheet leg section (32), a second portion (28b) of the first top sheet section (28) overlying the second back sheet waist section (30) and waist elastic strands (24) secured between the second back sheet waist section (30) and the second portion (28b) of the first top sheet section (28);
   a web guide assembly (76) arranged to spread the first and second welded strips (62,64) so that positions of the second back sheet waist section (30) and the back sheet leg section (32) extending in a third feed direction (F₃) are swapped relative to the first back sheet waist section (26) in a cross machine direction (X) such that the back sheet leg section (32) is located between the first and second back sheet waist sections (26,30);
   a leg elastic infeed assembly (80) to insert leg elastic strands (36) in a non-linear pattern in the third feed direction (F₃) on to the back sheet leg section (32) of the first continuous web (50); and
   a leg top sheet infeed (90) to direct the second top sheet section (34) to overlie the back sheet leg section (32) and sandwich the leg elastic strands (36) therebetween.
2. Apparatus (100) according to Paragraph 1 wherein the waist elastic infeed (68) includes one or more waist elastic feed elements to insert the waist elastic strands (24) to extend in substantially straight lines in the first feed direction (F₁) on to the first and second back sheet waist sections (26,30) with adjacent strands being spaced from each other in the cross-machine direction (X).
3. Apparatus (100) according to Paragraph 2 wherein the waist elastic feed element(s) insert the waist elastic strands (24) equidistantly spaced from each other in the cross-machine direction (X).
4. Apparatus (100) according to any one of Paragraphs 1-3 wherein the leg elastic infeed assembly (80) includes one or more reciprocating leg elastic feed elements to insert the leg elastic strands (24) in a periodic wave pattern in the third feed direction (F₃) on to the back sheet leg section (32).
5. Apparatus (100) according to any one of Paragraphs 1-3 wherein the leg elastic infeed assembly (80) includes a reciprocating drive element to drive movement of the back sheet leg section (32) relative to one or more leg elastic feed elements to insert the leg elastic strands (24) in a periodic wave pattern in the third feed direction (F₃) on to the back sheet leg section (32).
6. Apparatus (100) according to any one of Paragraphs 1-5 further including an adhesive assembly (84) configured to apply adhesive (38) to one or both of the back sheet leg section (32) and the leg elastic strands (36) to adhere the leg elastic strands (36) in the non-linear pattern between the back sheet leg section (32) and the second top sheet section (34).
7. Apparatus (100) according to any one of Paragraphs 1-5 further including a mechanical bonding mechanism configured to mechanically secure the leg elastic strands (36) in the non-linear pattern between the back sheet leg section (32) and the second top sheet section (34) absent adhesive.
8. Apparatus (100) according to any one of Paragraphs 1-7 wherein the apparatus (100) further includes a belly elastic infeed (82) to insert belly elastic strands (40) extending under tension in the third feed direction (F₃) on to the back sheet leg section (32), wherein the leg top sheet infeed (90) directs the second top sheet section (34) to overlie the back sheet leg section (32) and sandwich the leg elastic strands (36) and the belly elastic strands (40) therebetween.
9. Apparatus (100) according to Paragraphs 6 and 8 wherein the adhesive assembly (84) is further configured to apply adhesive (38) to one or both of the belly elastic strands (40) and the back sheet leg section (32) to adhere the belly elastic strands (40) between the back sheet leg section (32) and the second top sheet section (34).
10. Apparatus (100) according to Paragraphs 7 and 8 wherein the mechanical bonding mechanism is configured to mechanically secure the leg elastic strands (36) and the belly elastic strands (40) between the back sheet leg section (32) and the second top sheet section (34) absent adhesive.
11. Apparatus (100) according to any one of Paragraphs 8-10 wherein the belly elastic infeed (82) is located to insert belly elastic strands (40) extending under tension in the third feed direction (F₃) on to the back sheet leg section (32) at the same location as the insertion of the leg elastic strands (36).
12. Apparatus (100) according to any one of Paragraphs 8-11 wherein the belly elastic infeed apparatus (82) includes one or more belly elastic feed elements to insert the belly elastic strands (40) to extend in substantially straight lines along the back sheet leg section (32) in the third feed direction (F₃).
13. Apparatus (100) according to Paragraph 12 wherein the belly elastic feed element(s) insert the belly elastic strands (40) equidistantly spaced from each other in the cross-machine direction (X).
14. Apparatus (100) according to any one of Paragraphs 1-13 wherein the welding assembly (74) includes at least one rotary or blade-style ultrasonic horn (73) with a smooth or grooved working surface (77) and at least one opposing anvil (75) having a working surface (79) defining a plurality of raised ridges (81), each ridge (81) defining a smooth or grooved contact surface (83), the horn(s) (73) and anvil(s) (75) being arranged on opposing sides of a feed path through the welding assembly (74) so that the waist elastic strands (24) sandwiched between the first and second back sheet waist sections (26,30) and the first top sheet section (28a) travel in the first feed direction (F₁) between the horn(s) (73) and anvil(s) (75).
15. Apparatus (100) according to Paragraph 14 wherein the welding assembly (74) includes at least one blade-style ultrasonic horn (73) with a grooved working surface (77) and at least one opposing anvil (75) having a working surface (79) defining a plurality of raised ridges (81), each ridge defining a smooth contact surface (83).
16. Apparatus (100) according to Paragraph 14 or Paragraph 15 wherein each of the plurality of raised ridges (81) is non-linear in shape and the ridges (81) are arranged to define a repeating pattern.
17. Apparatus (100) according to Paragraph 16 wherein the repeating pattern of ridges (81) on the working surface (79) of the anvil(s) (75) is positioned so that a peak (85) in each of the ridges (81) is aligned with the or one of the ultrasonic horns (73) and the juncture between the first and second back sheet waist sections (26,30) of the first continuous web (50).
18. Apparatus (100) according to Paragraph 16 wherein the second slitting assembly (62) is positioned so that the second cut line (56) is aligned with a peak (85) in each of the ridges (81) on the working surface (79) of the anvil(s) (75) and the or one of the ultrasonic horns (73).
19. Apparatus (100) according to any one of Paragraphs 1-18 wherein the web guide assembly (76) includes a first pair of turnbars (102a,102b), each positioned at a first angle to longitudinal and transverse axes of a respective one of the first and second welded strips (62,64), and offset relative to each other along the longitudinal direction of travel of the first and second welded strips (62,64), so that on wrapping around the first pair of turnbars (102a,102b) the first and second welded strips (62,64) are inverted, with one being inverted upstream from the other.
20. Apparatus (100) according to Paragraph 19 wherein the first pair of turnbars (102a,102b) are defined by a single turnbar (103).
21. Apparatus (100) according to Paragraph 19 or Paragraph 20 wherein:
   the web guide assembly (76) further includes a second pair of turnbars (104a,104b) located immediately upstream or downstream of the first pair of turnbars (102a,102b), each positioned at a second angle relative to longitudinal and transverse axes of a respective one of the first and second welded strips (62,64), and offset from each other along the longitudinal direction of travel of the first and second welded strips (62,64), so that on wrapping around the second pair of turnbars (104a,104b) the first and second welded strips (62,64) are inverted, with one of the first and second welded strips (62,64) being inverted upstream from the other; and
   the first and second angles, and the offset between the turnbars in each of the first and second pairs of turnbars (102a,102b; 104a,104b) are selected so that on wrapping around the first and second pairs of turnbars (102a,102b;104a,104b) the positions of the second back sheet waist section (30) and the back sheet leg section (32) extending in the third feed direction (F₃) are swapped relative to the first back sheet waist section (26) in the cross-machine direction such that the back sheet leg section (32) is located between the first and second back sheet waist sections (26,30).
22. Apparatus (100) according to Paragraph 21 wherein the web guide assembly (76) further includes a pair of idler rollers (106a,106b) located between the first and second pairs of turnbars (102a,102b;104a,104b) and spaced orthogonally from each other relative to the longitudinal direction of travel of the first and second welded strips (62,64) so that on wrapping around each of the idler rollers (106a,106b) each of the first and second welded strips (62,64) follows a stepped feed path between the first and second pairs of turnbars (102a,102b;104a,104b).
23. Apparatus according to Paragraph 21 wherein the web guide assembly (76) further includes at least one idler roller (108) located between the first and second pairs of turnbars (102a,102b;104a,104b) so that on wrapping around the idler roller (108) the first and second welded strips (62,64) are inverted.
24. Apparatus (100) according to any one of Paragraphs 1-18 wherein the web guide assembly (76) includes two pairs of idler rollers (110a,110b) spaced from each other and angled relative to the first direction of travel (F₁) so that on passing around a respective one of the idler rollers pairs (110a,110b) the direction of travel of each of the first and second welded strips (62,64) is changed and the positions of the first and second welded strips (62,64) extending in the third feed direction (F₃) downstream of the web guide assembly (76) are swapped relative to each other in the cross machine direction (X) such that the back sheet leg section (32) is located between the first and second back sheet waist sections (26,30).
25. A method of manufacturing torso encircling portions (12,14) for use in the manufacture of pant-style diaper products comprising the steps of:
   operating a first web infeed assembly (51) to feed a first continuous web (50) generally in a first feed direction (F₁), the first continuous web (50) defining first and second back sheet waist sections (26,30) and a back sheet leg section (32) extending side by side in the first feed direction (F₁) with the second back sheet waist section (30) located between the first back sheet waist section (26) and the back sheet leg section (32);
   operating a second web infeed assembly (53) to feed a second continuous web (52) generally in a second feed direction (Fz), the second continuous web (52) defining first and second top sheet sections (28,34) extending side by side in the second feed direction (Fz);
   operating a first slitting assembly (60) to slit the second continuous web (52) along a first cut line (54) extending in the second feed direction (F₂) to separate the first and second top sheet sections (28,34);
   operating a waist elastic infeed (68) to insert waist elastic strands (24) under tension in the first feed direction (F₁) on to the first and second back sheet waist sections (26,30) of the first continuous web (50);
   operating a waist top sheet infeed (70) to direct the first top sheet section (28) to overlie the first and second back sheet waist sections (26,30) and sandwich the waist elastic strands (24) therebetween;
   operating a welding assembly (74) to weld the first top sheet section (28) and the first and second back sheet waist sections (26,30) together intermittently in the first feed direction (F₁) so as to entrap and secure the waist elastic strands (24) therebetween and form a welded structure (83);
   operating a second slitting assembly (66) to slit the welded structure (83) along a second cut line (56) extending in the first feed direction (F₁) to separate the welded structure (83) along a juncture between the first and second back sheet waist sections (26,30) of the first continuous web (50) and thereby define first and second welded strips (62,64), the first welded strip (62) including the first back sheet waist section (26), a first portion (28a) of the first top sheet section (28) and waist elastic strands (24) secured therebetween, and the second welded strip (64) including the second back sheet waist section (30), the back sheet leg section (32), a second portion (28b) of the first top sheet section (28) overlying the second back sheet waist section (30) and waist elastic strands (24) secured between the second back sheet waist section (30) and the second portion (28b) of the first top sheet section (28);
   operating a web guide assembly (76) to spread the first and second welded strips (62,64) so that positions of the second back sheet waist section (30) and the back sheet leg section (32) extending in a third feed direction (F₃) are swapped relative to the first back sheet waist section (26) in a cross-machine direction (X) such that the back sheet leg section (32) is located between the first and second back sheet waist sections (26,30);
   operating a leg elastic infeed assembly (80) to insert leg elastic strands (36) in a non-linear pattern in the third feed direction (F₃) on to the back sheet leg section (32) of the first continuous web (50); and
   operating a leg top sheet infeed (90) to direct the second top sheet section (34) to overlie the back sheet leg section (32) and sandwich the leg elastic strands (36) therebetween.
26. The method according to Paragraph 25 wherein the step of operating the waist elastic infeed (68) includes inserting the waist elastic strands (24) via one or more waist elastic feed elements so that the waist elastic strands (24) extend in substantially straight lines in the first feed direction (F₁) on to the first and second back sheet waist sections (26,30) with adjacent strands being spaced from each other in the cross-machine direction (X).
27. The method according to Paragraph 26 wherein the waist elastic strands (24) inserted via the waist elastic feed elements are equidistantly spaced from each other in the cross-machine direction (X).
28. The method according to any one of Paragraphs 25-27 wherein the step of operating the leg elastic infeed assembly (80) includes reciprocating one or more leg elastic feed elements to insert the leg elastic strands (24) in a periodic wave pattern in the third feed direction (F₃) on to the back sheet leg section (32).
29. The method according to any one of Paragraphs 25-27 wherein the step of operating the leg elastic infeed assembly (80) includes reciprocating a drive element to drive movement of the back sheet leg section (32) relative to one or more leg elastic feed elements to insert the leg elastic strands (24) in a periodic wave pattern in the third feed direction (F₃) on to the back sheet leg section (32).
30. The method according to any one of Paragraphs 25-29 further including the step of operating an adhesive assembly (84) to apply adhesive (38) to one or both of the back sheet leg section (32) and the leg elastic strands (36) to adhere the leg elastic strands (36) in the non-linear pattern between the back sheet leg section (32) and the second top sheet section (34).
31. The method according to any one of Paragraphs 25-29 further including the step of operating a mechanical bonding mechanism to mechanically secure the leg elastic strands (36) in the non-linear pattern between the back sheet leg section (32) and the second top sheet section (34) absent adhesive.
32. The method according to any one of Paragraphs 25-31 further including the step of operating a belly elastic infeed (82) to insert belly elastic strands (40) extending under tension in the third feed direction (F₃) on to the back sheet leg section (32), wherein the step of operating the leg top sheet infeed (90) includes directing the second top sheet section (34) to overlie the back sheet leg section (32) and sandwich the leg elastic strands (36) and the belly elastic strands (40) therebetween.
33. The method according to Paragraphs 30 and 32 wherein adhesive (38) is applied to one or both of the belly elastic strands (40) and the back sheet leg section (32) to adhere the belly elastic strands (40) between the back sheet leg section (32) and the second top sheet section (34).
34. The method according to Paragraphs 31 and 32 the leg elastic strands (36) and the belly elastic strands (40) are mechanically secured between the back sheet leg section (32) and the second top sheet section (34) absent adhesive.
35. The method according to any one of Paragraphs 32-34 wherein the belly elastic strands (40) are inserted, extending under tension in the third feed direction (F₃) on to the back sheet leg section (32) at the same location as the insertion of the leg elastic strands (36).
36. The method according to any one of Paragraphs 32-35 wherein the belly elastic strands are inserted via one or more belly elastic feed elements to extend in substantially straight lines along the back sheet leg section (32) in the third feed direction (F₃).
37. The method according to Paragraph 36 wherein the belly elastic strands (40) inserted via the belly elastic feed element(s) are equidistantly spaced from each other in the cross-machine direction (X).
38. The method according to any one of Paragraphs 25-37 wherein the step of welding the first top sheet section (28), the waist elastic strands (24) and the first and second back sheet waist sections (26,30) includes feeding the waist elastic strands (24) sandwiched between the first top sheet section (28) and the first and second back sheet waist sections (26,30) between at least one rotary or blade-style ultrasonic horn (73) with a smooth or grooved working surface (77) and at least one opposing anvil (75) having a working surface (79) defining a plurality of raised ridges (81), each ridge defining a smooth or grooved contact surface (83), and welding the first and second back sheet waist sections (26,30) to the first top sheet section (28) intermittently in the first feed direction (F₁) so as to entrap and secure the waist elastic strands therebetween and form the welded structure (87).
39. The method according to Paragraph 38 wherein the step of welding the first top sheet section (28), the waist elastic strands (24) and the first and second back sheet waist sections (26,30) includes feeding the waist elastic strands (24) sandwiched between the first top sheet section (28) and the first and second back sheet waist sections (26,30) between at least one blade-style ultrasonic horn (73) with a grooved working surface (77) and at least one opposing anvil (75) having a working surface (79) defining a plurality of raised ridges (81), each ridge (81) defining a smooth contact surface (83).
40. The method according to any one of Paragraphs 38 or Claim 39 wherein each of the plurality of raised ridges (81) is non-linear in shape and the ridges (81) are arranged to define a repeating pattern.
41. The method according to Paragraph 40 wherein the repeating pattern of ridges (81) on the working surface (79) of the anvil(s) (75) is positioned so that a peak (85) in each of the ridges (81) is aligned with the or one of the ultrasonic horns (73) and the juncture between the first and second back sheet waist sections (26,30) of the first continuous web (50).
42. The method according to Paragraph 40 wherein the second slitting assembly (62) is positioned so that the second cut line (56) is aligned with a peak (85) in each of the ridges (81) on the working surface (79) of the anvil(s) (75) and the or one of the ultrasonic horns (73).
43. The method according to any one of Paragraphs 25-42 wherein the step of spreading the first and second welded strips (62,64) includes wrapping each of the first and second welded strips (62,64) around a respective one of a first pair of turnbars (102a,102b) positioned at a first angle to longitudinal and transverse axes of the respective one of the first and second welded strips (62,64), and offset relative to each other along the longitudinal direction of travel of the first and second welded strips (62,64), so that the first and second welded strips (62,64) are inverted, with one being inverted upstream from the other.
44. The method according to Paragraph 43 wherein the first pair of turnbars (102a,102b) are defined by a single turnbar (103).
45. The method according to Paragraph 43 or Paragraph 44 wherein:
   the step of spreading the first and second welded strips (62,64) further includes wrapping each of the first and second welded strips (62,64) around a respective one of a second pair of turnbars (104a,104b) located immediately upstream or downstream of the first pair of turnbars (102a,102b) and positioned at a second angle to longitudinal and transverse axes of a respective one of the first and second welded strips (62,64), and offset from each other along the longitudinal direction of travel of the first and second welded strips (62,64), so that the one of the first and second welded strips (62,64) is inverted upstream from the other; and
   the first and second angles, and the offset between the turnbars in each of the first and second pairs of turnbars (102a,102b; 104a,104b) are selected so that on wrapping around the first and second pairs of turnbars (102a,102b;104a,104b) the positions of the second back sheet waist section (30) and the back sheet leg section (32) extending in the third feed direction (F₃) are swapped relative to the first back sheet waist section (26) in the cross-machine direction such that the back sheet leg section (32) is located between the first and second back sheet waist sections (26,30).
46. The method according to Paragraph 45 wherein the step of spreading the first and second welded strips (62,64) further includes wrapping the first and second welded strips (62,64) around a respective one a pair of idler rollers (106a,106b) located between the first and second pairs of turnbars (102a,102b;104a,104b) and spaced orthogonally from each other relative to the longitudinal direction of travel of the first and second welded strips (62,64) so that each of the first and second welded strips (62,64) follows a stepped path between the first and second pairs of turnbars (102a,102b;104a,104b).
47. The method according to Paragraph 45 wherein the step of spreading the first and second welded strips (62,64) further includes wrapping the first and second welded strips (62,64) around at least one idler roller (108) located between the first and second pairs of turnbars (102a,102b; 104a,104b) so that the first and second welded strips (62,64) are inverted.
48. The method according to any one of Paragraphs 25-42 wherein the step of spreading the first and second welded strips (62,64) includes feeding each of the first and second welded strips (62,64) around a respective one of the idler roller pairs (110a,110b) spaced from each other and angled relative to the first direction of travel (F₁) so that the direction of travel of each of the first and second welded strips (62,64) is changed and the positions of the first and second welded strips (62,64) extending in the third feed direction (F₃) downstream of the web guide assembly (76) are swapped relative to each other in the cross machine direction (X) such that the back sheet leg section (32) is located between the first and second back sheet waist sections (26,30).

## Claims

1. Apparatus (100) for manufacturing torso encircling portions (12,14) of a pant-style diaper product, the apparatus (100) comprising:
a first web infeed assembly (51) to feed a first continuous web (50) generally in a first feed direction (F₁), the first continuous web (50) defining first and second back sheet waist sections (26,30) and a back sheet leg section (32) extending side by side in the first feed direction (F₁) with the second back sheet waist section (30) located between the first back sheet waist section (26) and the back sheet leg section (32);
a second web infeed assembly (53) to feed a second continuous web (52) generally in a second feed direction (Fz), the second continuous web (52) defining first and second top sheet sections (28,34) extending side by side in the second feed direction (Fz);
a first slitting assembly (60) configured to slit the second continuous web (52) along a first cut line (54) extending in the second feed direction (F₂) to separate the first and second top sheet sections (28,34);
a waist elastic infeed (68) configured to insert waist elastic strands (24) under tension in the first feed direction (F₁) on to the first and second back sheet waist sections (26,30) of the first continuous web (50);
a waist top sheet infeed (70) configured to direct the first top sheet section (28) to overlie the first and second back sheet waist sections (26,30) and sandwich the waist elastic strands (24) therebetween;
a welding assembly (74) configured to weld the first top sheet section (28) and the first and second back sheet waist sections (26,30) together intermittently in the first feed direction (F₁) so as to entrap and secure the waist elastic strands (24) therebetween and form a welded structure (83);
a second slitting assembly (62) configured to slit the welded structure (83) along a second cut line (56) extending in the first feed direction (F₁) to separate the welded structure (83) along a juncture between the first and second back sheet waist sections (26,30) of the first continuous web (50) and thereby define first and second welded strips (62,64), the first welded strip (62) including the first back sheet waist section (26), a first portion (28a) of the first top sheet section (28) and waist elastic strands (24) secured therebetween, and the second welded strip (64) including the second back sheet waist section (30), the back sheet leg section (32), a second portion (28b) of the first top sheet section (28) overlying the second back sheet waist section (30) and waist elastic strands (24) secured between the second back sheet waist section (30) and the second portion (28b) of the first top sheet section (28);
a web guide assembly (76) arranged to spread the first and second welded strips (62,64) so that positions of the second back sheet waist section (30) and the back sheet leg section (32) extending in a third feed direction (F₃) are swapped relative to the first back sheet waist section (26) in a cross machine direction (X) such that the back sheet leg section (32) is located between the first and second back sheet waist sections (26,30);
a leg elastic infeed assembly (80) to insert leg elastic strands (36) in a non-linear pattern in the third feed direction (F₃) on to the back sheet leg section (32) of the first continuous web (50); and
a leg top sheet infeed (90) to direct the second top sheet section (34) to overlie the back sheet leg section (32) and sandwich the leg elastic strands (36) therebetween.

2. Apparatus (100) according to Claim 1 wherein the leg elastic infeed assembly (80) includes one or more reciprocating leg elastic feed elements to insert the leg elastic strands (24) in a periodic wave pattern in the third feed direction (F₃) on to the back sheet leg section (32).

3. Apparatus (100) according to Claim 1 or Claim 2 wherein the leg elastic infeed assembly (80) includes a reciprocating drive element to drive movement of the back sheet leg section (32) relative to one or more leg elastic feed elements to insert the leg elastic strands (24) in a periodic wave pattern in the third feed direction (F₃) on to the back sheet leg section (32).

4. Apparatus (100) according to any one of the preceding claims further including an adhesive assembly (84) configured to apply adhesive (38) to one or both of the back sheet leg section (32) and the leg elastic strands (36) to adhere the leg elastic strands (36) in the non-linear pattern between the back sheet leg section (32) and the second top sheet section (34).

5. Apparatus (100) according to any one of Claims 1-4 further including a mechanical bonding mechanism configured to mechanically secure the leg elastic strands (36) in the non-linear pattern between the back sheet leg section (32) and the second top sheet section (34) absent adhesive.

6. Apparatus (100) according to any one of the preceding claims wherein the apparatus (100) further includes a belly elastic infeed (82) to insert belly elastic strands (40) extending under tension in the third feed direction (F₃) on to the back sheet leg section (32), wherein the leg top sheet infeed (90) directs the second top sheet section (34) to overlie the back sheet leg section (32) and sandwich the leg elastic strands (36) and the belly elastic strands (40) therebetween.

7. Apparatus (100) according to Claims 4 and 6 wherein the adhesive assembly (84) is further configured to apply adhesive (38) to one or both of the belly elastic strands (40) and the back sheet leg section (32) to adhere the belly elastic strands (40) between the back sheet leg section (32) and the second top sheet section (34).

8. Apparatus (100) according to Claims 5 and 6 wherein the mechanical bonding mechanism is configured to mechanically secure the leg elastic strands (36) and the belly elastic strands (40) between the back sheet leg section (32) and the second top sheet section (34) absent adhesive.

9. Apparatus (100) according to any one of Claims 6-8 wherein the belly elastic infeed (82) is located to insert belly elastic strands (40) extending under tension in the third feed direction (F₃) on to the back sheet leg section (32) at the same location as the insertion of the leg elastic strands (36).

10. Apparatus (100) according to any one of Claims 6-9 wherein the belly elastic infeed apparatus (82) includes one or more belly elastic feed elements to insert the belly elastic strands (40) to extend in substantially straight lines along the back sheet leg section (32) in the third feed direction (F₃).

11. Apparatus (100) according to any one of the preceding claims wherein the welding assembly (74) includes at least one rotary or blade-style ultrasonic horn (73) with a smooth or grooved working surface (77) and at least one opposing anvil (75) having a working surface (79) defining a plurality of raised ridges (81), each ridge (81) defining a smooth or grooved contact surface (83), the horn(s) (73) and anvil(s) (75) being arranged on opposing sides of a feed path through the welding assembly (74) so that the waist elastic strands (24) sandwiched between the first and second back sheet waist sections (26,30) and the first top sheet section (28a) travel in the first feed direction (F₁) between the horn(s) (73) and anvil(s) (75).

12. Apparatus (100) according to Claim 11 wherein the welding assembly (74) includes at least one blade-style ultrasonic horn (73) with a grooved working surface (77) and at least one opposing anvil (75) having a working surface (79) defining a plurality of raised ridges (81), each ridge defining a smooth contact surface (83).

13. Apparatus (100) according to Claim 11 or Claim 12 wherein each of the plurality of raised ridges (81) is non-linear in shape and the ridges (81) are arranged to define a repeating pattern.

14. Apparatus (100) according to Claim 13 wherein the repeating pattern of ridges (81) on the working surface (79) of the anvil(s) (75) is positioned so that a peak (85) in each of the ridges (81) is aligned with the or one of the ultrasonic horns (73) and the juncture between the first and second back sheet waist sections (26,30) of the first continuous web (50).

15. Apparatus (100) according to Claim 13 wherein the second slitting assembly (62) is positioned so that the second cut line (56) is aligned with a peak (85) in each of the ridges (81) on the working surface (79) of the anvil(s) (75) and the or one of the ultrasonic horns (73).

16. Apparatus (100) according to any one of the preceding claims wherein the web guide assembly (76) includes a first pair of turnbars (102a,102b), each positioned at a first angle to longitudinal and transverse axes of a respective one of the first and second welded strips (62,64), and offset relative to each other along the longitudinal direction of travel of the first and second welded strips (62,64), so that on wrapping around the first pair of turnbars (102a,102b) the first and second welded strips (62,64) are inverted, with one being inverted upstream from the other.

17. Apparatus (100) according to Claim 16 wherein the first pair of turnbars (102a,102b) are defined by a single turnbar (103).

18. Apparatus (100) according to Claim 16 or Claim 17 wherein:
the web guide assembly (76) further includes a second pair of turnbars (104a,104b) located immediately upstream or downstream of the first pair of turnbars (102a,102b), each positioned at a second angle relative to longitudinal and transverse axes of a respective one of the first and second welded strips (62,64), and offset from each other along the longitudinal direction of travel of the first and second welded strips (62,64), so that on wrapping around the second pair of turnbars (104a,104b) the first and second welded strips (62,64) are inverted, with one of the first and second welded strips (62,64) being inverted upstream from the other; and
the first and second angles, and the offset between the turnbars in each of the first and second pairs of turnbars (102a,102b; 104a,104b) are selected so that on wrapping around the first and second pairs of turnbars (102a,102b;104a,104b) the positions of the second back sheet waist section (30) and the back sheet leg section (32) extending in the third feed direction (F₃) are swapped relative to the first back sheet waist section (26) in the cross-machine direction such that the back sheet leg section (32) is located between the first and second back sheet waist sections (26,30).

19. Apparatus (100) according to Claim 18 wherein the web guide assembly (76) further includes a pair of idler rollers (106a,106b) located between the first and second pairs of turnbars (102a,102b; 104a,104b) and spaced orthogonally from each other relative to the longitudinal direction of travel of the first and second welded strips (62,64) so that on wrapping around each of the idler rollers (106a,106b) each of the first and second welded strips (62,64) follows a stepped feed path between the first and second pairs of turnbars (102a,102b;104a,104b).

20. Apparatus according to Claim 18 wherein the web guide assembly (76) further includes at least one idler roller (108) located between the first and second pairs of turnbars (102a,102b;104a,104b) so that on wrapping around the idler roller (108) the first and second welded strips (62,64) are inverted.

21. Apparatus (100) according to any one of Claims 1-15 wherein the web guide assembly (76) includes two pairs of idler rollers (110a,110b) spaced from each other and angled relative to the first direction of travel (F₁) so that on passing around a respective one of the idler roller pairs (110a,110b) the direction of travel of each of the first and second welded strips (62,64) is changed and the positions of the first and second welded strips (62,64) extending in the third feed direction (F₃) downstream of the web guide assembly (76) are swapped relative to each other in the cross machine direction (X) such that the back sheet leg section (32) is located between the first and second back sheet waist sections (26,30).

22. A method of manufacturing torso encircling portions (12,14) for use in the manufacture of pant-style diaper products comprising the steps of:
operating a first web infeed assembly (51) to feed a first continuous web (50) generally in a first feed direction (F₁), the first continuous web (50) defining first and second back sheet waist sections (26,30) and a back sheet leg section (32) extending side by side in the first feed direction (F₁) with the second back sheet waist section (30) located between the first back sheet waist section (26) and the back sheet leg section (32);
operating a second web infeed assembly (53) to feed a second continuous web (52) generally in a second feed direction (Fz), the second continuous web (52) defining first and second top sheet sections (28,34) extending side by side in the second feed direction (Fz);
operating a first slitting assembly (60) to slit the second continuous web (52) along a first cut line (54) extending in the second feed direction (F₂) to separate the first and second top sheet sections (28,34);
operating a waist elastic infeed (68) to insert waist elastic strands (24) under tension in the first feed direction (F₁) on to the first and second back sheet waist sections (26,30) of the first continuous web (50);
operating a waist top sheet infeed (70) to direct the first top sheet section (28) to overlie the first and second back sheet waist sections (26,30) and sandwich the waist elastic strands (24) therebetween;
operating a welding assembly (74) to weld the first top sheet section (28) and the first and second back sheet waist sections (26,30) together intermittently in the first feed direction (F₁) so as to entrap and secure the waist elastic strands (24) therebetween and form a welded structure (83);
operating a second slitting assembly (62) to slit the welded structure (83) along a second cut line (56) extending in the first feed direction (F₁) to separate the welded structure (83) along a juncture between the first and second back sheet waist sections (26,30) of the first continuous web (50) and thereby define first and second welded strips (62,64), the first welded strip (62) including the first back sheet waist section (26), a first portion (28a) of the first top sheet section (28) and waist elastic strands (24) secured therebetween, and the second welded strip (64) including the second back sheet waist section (30), the back sheet leg section (32), a second portion (28b) of the first top sheet section (28) overlying the second back sheet waist section (30) and waist elastic strands (24) secured between the second back sheet waist section (30) and the second portion (28b) of the first top sheet section (28);
operating a web guide assembly (76) to spread the first and second welded strips (62,64) so that positions of the second back sheet waist section (30) and the back sheet leg section (32) extending in a third feed direction (F₃) are swapped relative to the first back sheet waist section (26) in a cross-machine direction (X) such that the back sheet leg section (32) is located between the first and second back sheet waist sections (26,30);
operating a leg elastic infeed assembly (80) to insert leg elastic strands (36) in a non-linear pattern in the third feed direction (F₃) on to the back sheet leg section (32) of the first continuous web (50); and
operating a leg top sheet infeed (90) to direct the second top sheet section (34) to overlie the back sheet leg section (32) and sandwich the leg elastic strands (36) therebetween.
